(19) **Europäisches Patentamt / European Patent Office / Office européen des brevets**

(11) **EP 3 298 955 A1**

(12) # EUROPEAN PATENT APPLICATION

(43) Date of publication:
28.03.2018 Bulletin 2018/13

(51) Int Cl.:
*A61B 5/00* (2006.01)     *A61B 5/11* (2006.01)

(21) Application number: **17162231.9**

(22) Date of filing: **22.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.09.2016 IN 201621032626**

(71) Applicant: **Tata Consultancy Services Limited Maharashtra (IN)**

(72) Inventors:
• **Chakravarty, Kingshuk**
  **700160 Kolkata - West Bengal (IN)**
• **Sinha, Aniruddha**
  **700160 Kolkata - West Bengal (IN)**

• **Bhowmick, Brojeshwar**
  **700160 Kolkata - West Bengal (IN)**
• **Sinha, Sanjana**
  **700160 Kolkata - West Bengal (IN)**
• **Das, Abhijit**
  **700082 Kolkata - West Bengal (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

Remarks:
A request for correction of the drawings filed pursuant to Rule 139 EPC. A decision on the request will be taken during the proceedings before the Examining Division (Guidelines for Examination in the EPO, A-V, 3.).

(54) **METHOD AND SYSTEM FOR DETERMINING POSTURAL BALANCE OF A PERSON**

(57) A method and system for determining postural balance of the person is provided. The disclosure provides a single limb stance body balance analysis system which will aid medical practitioners to analyze crucial factor for fall risk minimization, injury prevention, fitness and rehabilitation. Skeleton data was captured using Kinect. Two parameters vibration-jitter and force per unit mass (FPUM) are derived for each body part to assess postural stability during SLS. Further, the vibration and force imposed on each joint a first balance score was quantified. A first balance score and a second balance are also calculated by combining vibration index and SLS duration to indicate the postural balance of the person. The vibration index is computed from the vibration profile associated different body segments.

FIG. 1

EP 3 298 955 A1

**Description**

CROSS-REFERENCE TO RELATED APPLICATIONS AND PRIORITY

This patent application claims priority to India Patent Application 201621032626, filed on September 23, 2016.

TECHNICAL FIELD

The present application generally relates to the field of monitoring postural balance of a person. More particularly, but not specifically, the invention provides a system and method for determining postural balance of the person in terms of balance scores calculated using the singles limb stance duration and vibration index of the joints of the person.

BACKGROUND OF THE INVENTION

Synchronized and coordinated activation of the postural muscles of the trunk and lower limbs is required for maintaining equilibrium and balance in human body. Poor postural balance control causes injury or falls in huge population and is supposed to be a critical factor of common motor skills. The patient who has survived the stroke disease are prone to fall due to imbalance. Therefore they require physiotherapy and other exercises. The determination of the postural balance of the person has become very critical, especially stroke survivors. The determination of the postural balance will help the caregiver to design the training and physiotherapy for the stroke survivors.

Several techniques already exist in the literature for measuring postural control in any stance. Among them Single Limb Stance (SLS) is a good option which not only assesses postural steadiness in a static position by a temporal measurement but also analyses the role of body joints in postural stability and correction. For clinicians, it provides a quick, reliable and easy way to screen their patients for fall risks and is easily incorporated into a comprehensive functional evaluation for older adults. SLS training for healthy subjects reduces chances of injury or fall by improving static balance. Being a complex mechanism, lack of postural control also creates postural sway during standing e.g. people with low back pain have been observed to have increased postural sway in standing.

Balance in SLS needs to be assessed in terms of both SLS-duration and body-sway which can be measured by center of pressure (COP) movement registered using stabilometry with force platforms. The COP reflects both the horizontal location of the center of gravity and the reaction forces due to muscular activity but does not inform about how postural perturbation creates instability/oscillation in different body parts/joints. Some works are reported where only amplitude of COP movements but omitted frequency associated with each joint vibration where clear relationship exists between the oscillation of COP and COM. Very recently in marker-based motion capture and analysis systems have been used for body sway measurement which is expensive, complex and the test can only be performed in the lab or clinic environment. In yet another study, the reliability of Kinect was analyzed for assessing the standing balance in terms of COM parameters but they did not discussed about body vibration during SLS in Euclidean coordinate x, y, z.

SUMMARY

The following presents a simplified summary of some embodiments of the disclosure in order to provide a basic understanding of the embodiments. This summary is not an extensive overview of the embodiments. It is not intended to identify key/critical elements of the embodiments or to delineate the scope of the embodiments. Its sole purpose is to present some embodiments in a simplified form as a prelude to the more detailed description that is presented below.

In view of the foregoing, an embodiment herein provides a system for determining postural balance of a person. The system comprises a 3D motion sensor, a noise filtering module, a memory and a processor in communication with the memory. The 3D motion sensor captures a skeleton data of the person. The person is performing a single limb stance (SLS) exercise. The noise filtering module removes a plurality of noises from the skeleton data. The processor further configured to perform the steps to: calculate a single limb stance (SLS) duration of the person using the skeleton data of the person; compute a velocity profile of each joints of the person; measure a vibration jitter and a force per unit mass (FPUM) of the person using the a joint movement profile of the person in 3D space, wherein the joint movement profile is different for each joints of the person, wherein the joint movement profile is obtained from the 3D motion sensor; generate a vibration index based on the vibration jitter and FPUM of the person for each of the joints; And generate a first balance score and a second balance score of the person using the SLS duration and the vibration index, wherein the first and the second balance score is indicative of the postural balance of the person.

Another embodiment provides a method for determining postural balance of a person, the method comprises various processor implemented steps as follows. Initially, a skeleton data of the person is obtained using a 3D motion sensor. The person is performing a single limb stance (SLS) exercise. The plurality of noises are then removed from the skeleton data using a noise filtering module. In the next step, a single limb stance (SLS) duration of the person is calculated using the skeleton data of the person. In the next step, a velocity profile of each joints of the person is computed. Further, a vibration jitter and a force per unit mass (FPUM) of the person is measured using a joint movement profile of the person in 3D space, wherein the joint movement profile is different for each joints of the person, wherein the joint movement profile is obtained from the 3D motion sensor. In the next step a vibration index is generated based on the vibration jitter and FPUM of the person for each of the joints. And finally, a first balance score and a second balance score of the person is generated using the SLS duration and the vibration index, wherein the first and the second balance score is indicative of the postural balance of the person.

BRIEF DESCRIPTION OF THE DRAWINGS

The embodiments herein will be better understood from the following detailed description with reference to the drawings, in which:

Fig. 1 illustrates a block diagram of a system for determining postural balance of a person, in accordance with an embodiment of the present disclosure;
Fig. 2 illustrates and experimental setup and various body joints which are monitored in the system in accordance with an embodiment of the present disclosure;
Fig. 3 shows the graphical representation of the variation of the left ankle of the person, in accordance with an embodiment of the disclosure;
Fig. 4 shows the graphical representation of curvature points identified k-mean clustering, in accordance with an embodiment of the disclosure; and
Fig. 5 is a flowchart illustrating the steps involved for determining postural balance of the person, in accordance with an embodiment of the disclosure.

DETAILED DESCRIPTION OF THE INVENTION

The embodiments herein and the various features and advantageous details thereof are explained more fully with reference to the non-limiting embodiments that are illustrated in the accompanying drawings and detailed in the following description. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

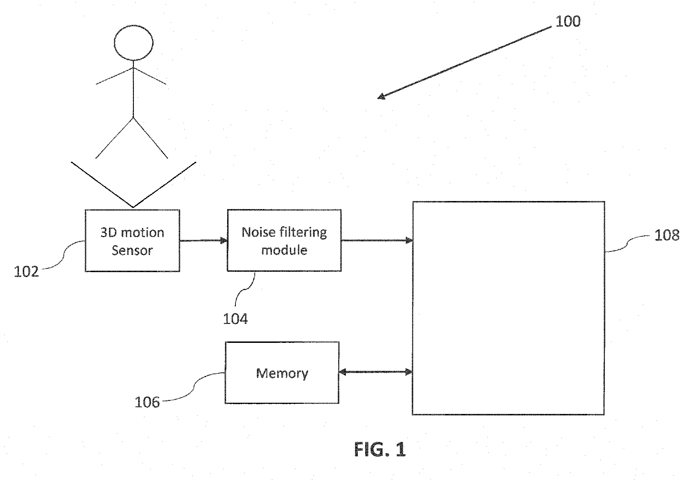

Referring now to the drawings, and more particularly to FIG. 1, where similar reference characters denote corresponding features consistently throughout the figures, there are shown preferred embodiments and these embodiments are described in the context of the following exemplary system and/or method.

According to an embodiment of the disclosure, a system 100 for determining postural balance of a person. The disclosure provides an unobtrusive system and method to compute the single limb stance (SLS) duration and body vibration in terms of 3D movements of joints of the person using only the skeleton data obtained from a 3D motion sensor such as Kinect. The vibration for each skeleton joint/body segment for single limb stance as well as bipedal stance phase is then used to generate balance scores based on the vibration index and SLS duration.

According to an embodiment of the disclosure, a block diagram of the system 100 is shown in Fig. 1. The system 100 includes a 3D motion sensor 102, a noise filtering module 104, memory 106 and a processor 108 in communication with the memory 106. The memory 106 is configured to store a plurality of algorithms. The processor 108 further includes a plurality of modules for performing various functions. The plurality of modules access the plurality of algorithms stored in the memory 106 to perform various functions.

According to an embodiment of the disclosure, a skeleton data of the person is obtained using the 3D motion sensor 102. In the present embodiment, Microsoft Kinect™ (Kinect) device has been used as the 3D motion sensor 102 for data capturing. It should be appreciated that for the sake of convenience to the reader, the word "3D motion sensor" and "Kinect" will be used replaceable in the disclosure. Microsoft's Kinect™ is a peripheral device that connects as an external interface to Microsoft's Xbox 360™ or to Microsoft Windows™ computers. The Kinect™ and the associated programmed computer or Xbox sense, recognize, and utilize the user's anthropomorphic form so the user can interact with software and media content without the need for a separate controller.

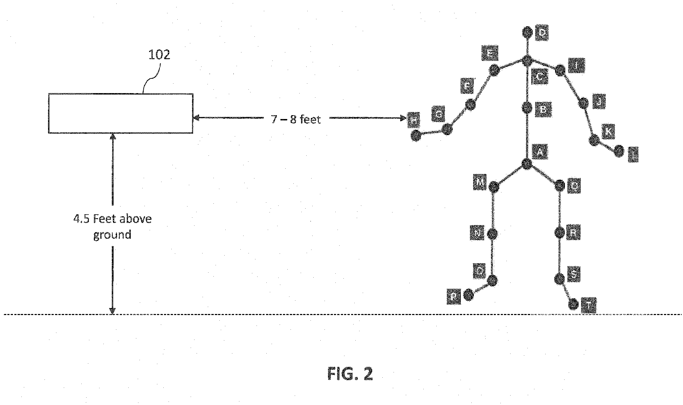

The experimental setup for capturing the skeleton data is shown in Fig. 2. In the embodiment, the person is asked to perform the single leg stance (SLS) exercise. The SLS exercise is performed with eyes open and arms on the hips and standing at 7-8 feet distance away from Kinect. While the participant must stand unassisted on one leg and is timed in seconds from the time one foot is flexed off the floor to the time when it touches the ground or the standing leg or an arm leaves the hips. The 3-D spatio-temporal information about 20 joints are obtained from Kinect. For ground truth, time synchronized data capture is carried out using Kinect and Force plate based setup. Subject's video can also be recorded to validate the experimental findings manually.

According to an embodiment of the disclosure, the system 100 also includes the noise filtering module 104. The skeleton data obtained from the 3D motion sensor 102 is very noisy and it is practically visible when the subject stands completely static, but some joints are moving in skeleton. There are many parameters that affect the characteristics and level of noise, which include, electromagnetic noise, room lighting, IR interference, quantization noise etc. It should be appreciated that the use of any existing noise filtering method is well within the scope of this disclosure.

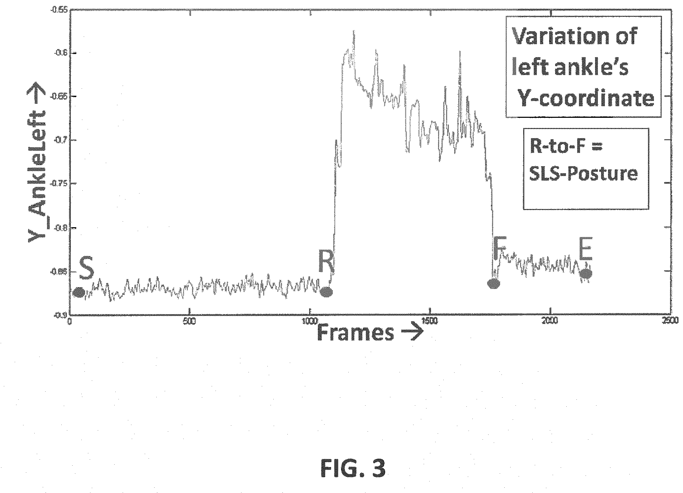

According to an embodiment of the disclosure, the processor 108 calculates an SLS duration of the person using the skeleton data of the person. During SLS exercise, variation in lifted leg's ankle coordinates is very much obvious. The skeleton joints obtained from the 3D motion sensor 102 are represented by 3D world co-ordinates (x,y,z) where 'x' represents left/right variation, 'y' represents up/down variation w.r.t ground and 'z' represents to/from variation of subject w.r.t the Kinect 102. So here, changes in the lifted leg's ankle y-co-ordinate (say, left leg is lifted) $Y_{AnkleLeft}$ gives meaningful information about the precise timing when a subject lifts leg (here, left-leg) above the ground. The same is shown in Fig. 3, which shows substantial change in $Y_{AnkleLeft}$ at point R, F and the zone R-to-F is the desired zone of SLS posture. In the other words, R is the frame where foot is flexed off the floor and F is the frame where it again touches the ground. The duration between R & F is considered as SLS duration. Keeping this fact in mind, k-means clustering algorithm is used to capture the variation in $Y_{AnkleLeft}$ with time. It helps in differentiating one leg stance portion (zone R-to-F). K-means mainly does the segregation (i.e. groups the data into 2 clusters) by optimizing following equation

$$O = \sum_{j=1}^{2} \sum_{i=1}^{N} \left\| Y^{(j)}_{AnkleLeft_i} - c_j \right\|, \quad c_j = \frac{1}{N} \sum_{j=1}^{N} (Y^{j}_{AnkleLeft_i})$$

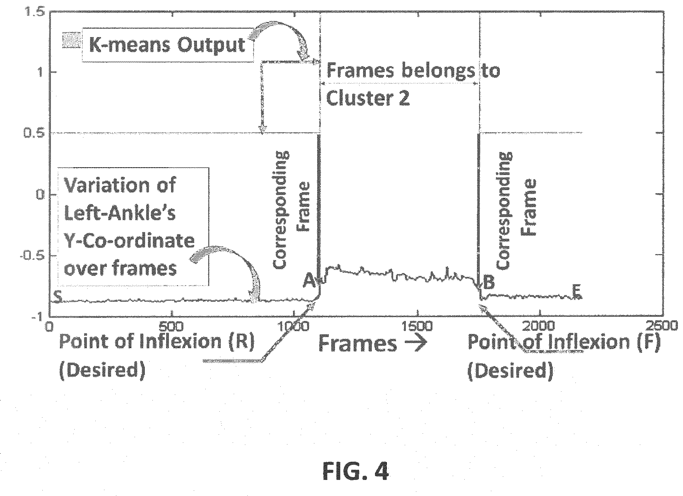

where $\|Y^{(j)}_{AnkleLefti} - c\|$ is a Euclidean distance between a data point $Y^{(j)}_{AnkleLefti}$ and the cluster center $c_j$. Frames belong to R-to-F will form one cluster, whereas rest will group into another one, as O is the indicator of the distance of the N data points from their respective cluster centres. Fig. 4 shows output of k-means algorithm i.e. frame A and B which are far away from the desired frames R and F. Consider data points X in region S-A. The fact that the curvature point will lie in the direction of minimum variance of data will be used further. So, compute covariance matrix $\hat{X}\hat{X}^T$ the mean subtracted data $\hat{X}$ is computed and the Eigen value decomposition of the matrix is also computed. This is the principle behind Principle Component Analysis (PCA) to find the direction of maximum variance. The eigenvector (say, $E_{min}$) corresponding to least eigenvalue provides the direction of minimum variance of the data and so reveals the direction towards curvature points. The curvature points R and F are obtained through minimum projection error of the Eigen vector corresponding to smallest Eigen-value using following equation:

$$\underset{r}{\operatorname{argmin}}[\vec{P} - (P.\hat{u})\hat{u}],$$

where $\bar{P}$ is the original signal value ($Y_{AnkleLeft}(r)$) at frame r (or time instance t); $\hat{u}$ is the unit vector along $\sim E_{min}$. Finally SLS duration is measured by finding difference between timestamps corresponding R and F frames.

According to an embodiment of the disclosure, the processor 018 further computes the velocity profile of each joints of the person. At the same time, a vibration jitter and a force per unit mass (FPUM) of the person is also measured by the processor 108 using a joint movement profile of the person in 3D space, wherein the joint movement profile is obtained from the 3D motion sensor 102. Generally, the joint movement profile is different for each joints of the person.

During the SLS exercise while standing on single limb, the person oscillates in order to maintain the balance. Moreover, for a given posture the person cannot move some of the joints like HipCenter, ShoulderCenter etc. easily and flexibly. Hence, the twenty different joints in the skeleton have different degree of freedom (DOF) e.g. it is high for hand but low for HipCenter. This DOF has strong impact on joint movement. To measure the oscillation quantitatively, velocity profile of each joint is used for its vibration analysis. Vibration is composed of frequency and amplitude. Higher frequency indicates more vibration and less balance. The velocity $\vec{Vel} = [v_x; v_y; v_z]$ in all the three directions viz. x, y, and z is analyzed for estimating the vibration or indirectly balance. The velocity is obtained from the filtered data using the following equation:

$$\vec{Vel} = [v_x^j,\ v_y^j,\ v_z^j] = \frac{d}{dt}[x^j, y^j, z^j]$$

where $x^j$, $y^j$, and $z^j$ is the displacement in (x; y; z) direction respectively for $j^{th}$ skeleton joint. It is observed from the AnkleLeft's velocity profile that velocity is maximum near R and minimum (considering sign) near F. Also the the mean velocity of AnkleLeft in first segment S-to-R is almost similar to the third one as shown in Fig. 3, whereas the velocity in the second is much higher than the other two. Start (R) and end (F) frames/time of one leg stance posture have already been identified previously. Hence, three different segments namely S-to-R (segment-1), R-to-F (segment-2) and F-to-E (segment-3) need to be analyzed separately. This fact is also true for all 20 joints. To get the information about frequency, every joint data in each segment is partitioned into a window of 50 samples and Fourier transform of each segment is evaluated following equation:

$$V_k^j(\omega) = \sum_{n=0}^{N-1} v_k^j[n]e^{-i\omega n},\ \ i^2 = -1;$$

where $V_k^j(\omega)$ is the frequency response of $i^{th}$ window for $j^{th}$ velocity $v_k^j$. This is done for all joints and in all three directions (x,y,z). Frequency $(f_k^j)$ corresponding to the maximum amplitude (Ajk) in each window is selected and the mean frequency of each segment is evaluated using following equation:

$$f_m^j = \frac{\sum A_k^j f_k^j}{\sum A_k^j}$$

Using above equation, mean frequencies $f_m^j\big|_{S-to-F}$, $f_m^j\big|_{R-to-F}$, $f_m^j\big|_{F-to-E}$ in each segment are computed. These calculated mean frequencies will eventually help us to analyze relative frequency variation (vibration) in corresponding segments i.e. before, during and after SLS. In this work, the relative frequency variation is considered as vibration-jitter (in Hz) and for each segment it is mathematically modeled using following equation:

$$J_{1,2,3} = (f_m^j - f_{\forall k}^j)\big|_{1,2,3}$$

where $J_{1,2,3}$ is vibration-jitter and $f_m^j$ mean frequency in each segment whereas $f_{\forall k}^j$ is the frequency for all windows in each segment. $J_{1,2,3}$ also quantifies vibration in terms of frequency for three segments, where more vibration indicates worse balance. For convenience, we will use the term jitter instead of vibration-jitter in rest of the disclosure. The dominant component of velocity for each window can be written as $v_k^j[n] = A_k^j cos(2\pi f_k^j n)$ where $f_k^j$ is the frequency corresponding to the maximum amplitude $A_k^j$ in $k^{th}$ window for jth joint of each segment.

It is evident from Biomechanics that during SLS, the force imposed on each joint to restore the equilibrium state is due to body weight, abductor muscles force and joint reaction force. This force can be a good measure for joint balance estimation. Keeping these facts in mind, the reaction force per unit mass (FPUM), FPUM = force / mass in meters/sec$^2$ for each joint is measured as the rate of change of velocity for that joint, i.e. acceleration (a). This can be better explained using Newton's law of motion i.e. F = ma

According to an embodiment of the disclosure, the processor 108 further configured to generate a first balance score and a second balance score of the person using the SLS duration and the vibration index. The first balance score and the second balance score is indicative of the postural balance of the person. These two scores are calculated using the SLS duration and the vibration index (VI - corrective body vibrations, measured quantitatively in terms of angular motion across hip, knee and trunk). Two type of scores, the first balance score and the second balance are computed as follows

The first balance score:

$$Score1 = 1 - \exp(-B*beta);$$

Where B = (1/VI).
Beta = 0.00490

The second balance score:

$$Score2 = 1 - \exp(-A*beta);$$

Where A = alpha * (SLS Duration) + (1-alpha) * (1/VI), and
Beta = 0.00490

In an embodiment of the dislcosure, the value of beta is taken as 0.004904 based on the dataset. Though it should be appreciated that the value of the beta may be different in another embodiment.

The first balance score is indicative of how much vibration is associated to different joint/body parts during single limb stance. It is a control score which indicates how long a subject can hold the single leg stance along with stability factor. The second balance score is indicative of the vibration in each joints in 3 dimensional space and the time duration for which the person stays in the single limb stance. According to another embodiment of the disclosure, the second balance score is validated with respect to the clinically approved scale such as Berg Balance Scale, Timed up and go. Regression analysis is performed to correlate these score with clinically approved scales.

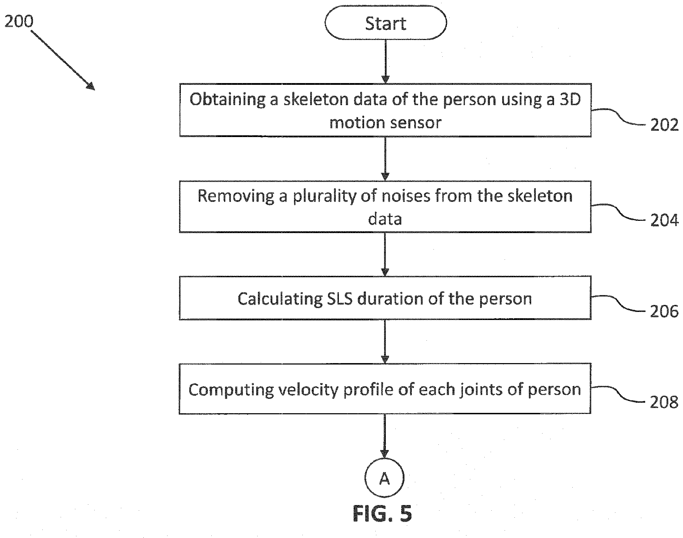

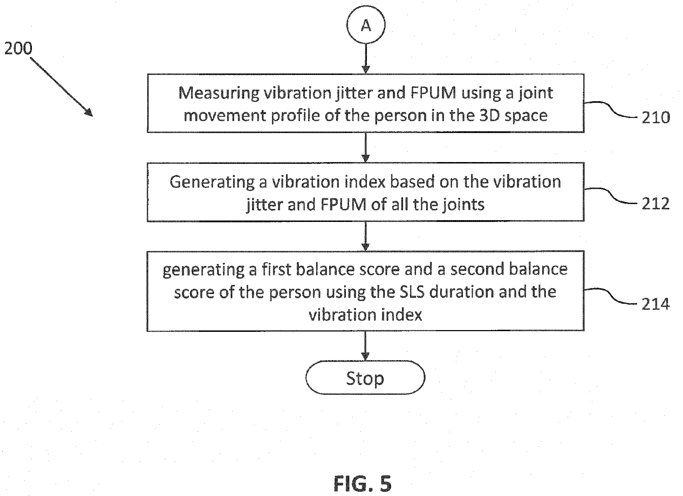

In operation, a flowchart 200 for determining postural balance of a person is shown in Fig. 5 according to an embodiment of the disclosure. Initially at step 202, the skeleton data of the person is obtained using the 3D motion sensor 102 such as the Kinect. At the time of data capturing the person is asked to perform the single limb stance (SLS) exercise in front of the stance as shown in the experimental setup of Fig. 2. At step 204, the plurality of noises such as electromagnetic noise, white noise etc. from the skeleton data are removed using the noise filtering module 104. In the next step 206, the single limb stance (SLS) duration of the person is measured using the skeleton data of the person. It should be appreciated that in an embodiment the SLS duration is measured using Eigen vector based curvature analysis method. Though use of any other method is well within the scope of this disclosure.

At step 208, the velocity profile of each joints of the person is measured. At step 210 the vibration jitter and a force per unit mass (FPUM) of the person is measured using a joint movement profile of the person in 3D space. The joint movement profile is obtained from the 3D motion sensor 102. In the next step 212, a vibration index (VI) is generated based on the vibration jitter and FPUM of the person. The Vibration index (VI) is an aggregated score comprising all joint's vibration profile. And finally at step 214, the first balance score and the second balance score of the person is generated using the SLS duration and the vibration index. The first and the second balance score jointly is indicative of the postural balance of the person. The second balance score is validated with respect to the clinically approved scale such as Berg Balance Scale, Timed up and go. Regression analysis is performed to correlate these score with clinically approved scales

According to an embodiment of the disclosure, the measured SLS duration, vibration jitter and FPUM can also be validated using a Brand-Altman plot. The Brand Altman plot provides a standard method to validate the experimental findings.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims. The embodiment, thus provides the system and method for determining the postural balance of the person in terms two balance scores.

It is, however to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk-read only memory (CD-ROM), compact disk-read/write (CD-R/W) and DVD.

A data processing system suitable for storing and/or executing program code will include at least one processor coupled directly or indirectly to memory elements through a system bus. The memory elements can include local memory employed during actual execution of the program code, bulk storage, and cache memories which provide temporary storage of at least some program code in order to reduce the number of times code must be retrieved from bulk storage during execution.

Input/output (I/O) devices (including but not limited to keyboards, displays, pointing devices, etc.) can be coupled to the system either directly or through intervening I/O controllers. Network adapters may also be coupled to the system to enable the data processing system to become coupled to other data processing systems or remote printers or storage devices through intervening private or public networks. Modems, cable modem and Ethernet cards are just a few of the currently available types of network adapters.

A representative hardware environment for practicing the embodiments may include a hardware configuration of an information handling/computer system in accordance with the embodiments herein. The system herein comprises at least one processor or central processing unit (CPU). The CPUs are interconnected via system bus to various devices such as a random access memory (RAM), read-only memory (ROM), and an input/output (I/O) adapter. The I/O adapter can connect to peripheral devices, such as disk units and tape drives, or other program storage devices that are readable by the system. The system can read the inventive instructions on the program storage devices and follow these instructions to execute the methodology of the embodiments herein.

The system further includes a user interface adapter that connects a keyboard, mouse, speaker, microphone, and/or other user interface devices such as a touch screen device (not shown) to the bus to gather user input. Additionally, a communication adapter connects the bus to a data processing network, and a display adapter connects the bus to a display device which may be embodied as an output device such as a monitor, printer, or transmitter, for example. The preceding description has been presented with reference to various embodiments. Persons having ordinary skill in the art and technology to which this application pertains will appreciate that alterations and changes in the described structures and methods of operation can be practiced without meaningfully departing from the principle, spirit and scope.

## Claims

1.  A method for determining postural balance of a person, the method comprising a processor implemented steps of:

> obtaining a skeleton data of the person using a 3D motion sensor, wherein the person is performing a single limb stance (SLS) exercise;
> removing a plurality of noises from the skeleton data using a noise filtering module;
> calculating a single limb stance (SLS) duration of the person using the skeleton data of the person;
> computing a velocity profile of each joints of the person;
> measuring a vibration jitter and a force per unit mass (FPUM) of the person using a joint movement profile of the person in 3D space, wherein the joint movement profile is different for each joints of the person, wherein the joint movement profile is obtained from the 3D motion sensor;
generating a vibration index based on the vibration jitter and FPUM of the person for each of the joints; and
generating a first balance score and a second balance score of the person using the SLS duration and the vibration index, wherein the first and the second balance score is indicative of the postural balance of the person.

2. The method of claim 1, wherein the first balance score is indicative of how much vibration is associated to different joint/body parts during single limb stance.

3. The method of claim 1, wherein the second balance score is indicative of the vibration in each joints in 3 dimensional space and the time duration for which the person stays in the single limb stance.

4. The method of claim 1 further comprising validating the second balance score with respect to a standard clinical score.

5. The method of claim 4, wherein the standard clinical score is at least one of a Berg Balance Scale or a Timed up and go scale

6. The method of claim 1, wherein the SLS duration of the person is measured using an Eigen vector based curvature analysis.

7. The method of claim 1, wherein the plurality of noises are at least one of an electromagnetic interference, IR interference, or a quantization noise.

8. The method of claim 1 further comprising validating the measured SLS duration, vibration jitter and FPUM using a Brand-Altman plot.

9. The method of claim 1 further comprising segmentation of the skeleton data in before the SLS exercise, during the SLS exercise and after the SLS exercise.

10. The method of claim 1, wherein the skeleton data is the measurement of the spatio-temporal variation of twenty joints of the person in the X-Y-Z coordinates.

11. A system for determining postural balance of a person, the system comprising:

a 3D motion sensor for obtaining a skeleton data of the person, wherein the person is performing a single limb stance (SLS) exercise;
a noise filtering module for removing a plurality of noises from the skeleton data;
a memory; and
a processor in communication with the memory, wherein the processor further configured to perform the steps to:

calculate a single limb stance (SLS) duration of the person using the skeleton data of the person;
compute a velocity profile of each joints of the person;
measure a vibration jitter and a force per unit mass (FPUM) of the person using the a joint movement profile of the person in 3D space, wherein the joint movement profile is obtained from the 3D motion sensor;
generate a vibration index based on the vibration jitter and FPUM of the person; and
generate a first balance score and a second balance score of the person using the SLS duration and the vibration index, wherein the first and the second balance score is indicative of the postural balance of the person.

12. The system of claim 11, wherein the 3D motion sensor is a Kinect sensor.

13. One or more non-transitory machine readable information storage mediums comprising one or more instructions which when executed by one or more hardware processors causes:

obtaining a skeleton data of the person using a 3D motion sensor, wherein the person is performing a single limb stance (SLS) exercise;
removing a plurality of noises from the skeleton data using a noise filtering module;
calculating a single limb stance (SLS) duration of the person using the skeleton data of the person;
computing a velocity profile of each joints of the person;

measuring a vibration jitter and a force per unit mass (FPUM) of the person using a joint movement profile of the person in 3D space, wherein the joint movement profile is different for each joints of the person, wherein the joint movement profile is obtained from the 3D motion sensor;

generating a vibration index based on the vibration jitter and FPUM of the person for each of the joints; and

generating a first balance score and a second balance score of the person using the SLS duration and the vibration index, wherein the first and the second balance score is indicative of the postural balance of the person.

14. The processor implemented method of claim 13, wherein the first balance score is indicative of how much vibration is associated to different joint/body parts during single limb stance.

15. The method of claim 13, wherein the second balance score is indicative of the vibration in each joints in 3 dimensional space and the time duration for which the person stays in the single limb stance.

16. The method of claim 13 further comprising validating the second balance score with respect to a standard clinical score.

17. The method of claim 13, wherein the SLS duration of the person is measured using an Eigen vector based curvature analysis.

18. The method of claim 13, wherein the plurality of noises are at least one of an electromagnetic interference, IR interference, or a quantization noise.

19. The method of claim 13 further comprising validating the measured SLS duration, vibration jitter and FPUM using a Brand-Altman plot.

20. The method of claim 13 further comprising segmentation of the skeleton data in before the SLS exercise, during the SLS exercise and after the SLS exercise.

FIG. 1

FIG. 2

**FIG. 3**

**FIG. 4**

200

```
           ┌─────────────┐
           │    Start    │
           └──────┬──────┘
                  │
                  ▼
┌──────────────────────────────────────────┐
│ Obtaining a skeleton data of the person   │── 202
│ using a 3D motion sensor                  │
└──────────────────┬───────────────────────┘
                   │
                   ▼
┌──────────────────────────────────────────┐
│ Removing a plurality of noises from the   │── 204
│ skeleton data                             │
└──────────────────┬───────────────────────┘
                   │
                   ▼
┌──────────────────────────────────────────┐
│ Calculating SLS duration of the person    │── 206
└──────────────────┬───────────────────────┘
                   │
                   ▼
┌──────────────────────────────────────────┐
│ Computing velocity profile of each joints │── 208
│ of person                                 │
└──────────────────┬───────────────────────┘
                   │
                   ▼
                  (A)
```

# FIG. 5

200

A

Measuring vibration jitter and FPUM using a joint movement profile of the person in the 3D space — 210

Generating a vibration index based on the vibration jitter and FPUM of all the joints — 212

generating a first balance score and a second balance score of the person using the SLS duration and the vibration index — 214

Stop

**FIG. 5**

Europäisches
Patentamt

European
Patent Office

Office européen
des brevets

## EUROPEAN SEARCH REPORT

Application Number

EP 17 16 2231

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | "Single leg stance (SLS) and vibration index (VI): New instrumental indices for fall risk estimation in stroke survivors", GAIT & POSTURE, ELSEVIER, AMSTERDAM, NL, vol. 49, 9 August 2016 (2016-08-09), page 168, XP029736673, ISSN: 0966-6362, DOI: 10.1016/J.GAITPOST.2016.07.224 * the whole document * | 1-20 | INV. A61B5/00 A61B5/11 |
| Y | CHAKRAVARTY KINGSHUK ET AL: "Quantification of balance in single limb stance using kinect", 2016 IEEE INTERNATIONAL CONFERENCE ON ACOUSTICS, SPEECH AND SIGNAL PROCESSING (ICASSP), IEEE, 20 March 2016 (2016-03-20), pages 854-858, XP032900722, DOI: 10.1109/ICASSP.2016.7471796 [retrieved on 2016-05-18] * the whole document * | 1-20 | |
| | | | TECHNICAL FIELDS SEARCHED (IPC) |
| A | ROSS A. CLARK ET AL: "Reliability and concurrent validity of the Microsoft Xbox One Kinect for assessment of standing balance and postural control", GAIT & POSTURE, vol. 42, no. 2, 1 July 2015 (2015-07-01), pages 210-213, XP055410408, AMSTERDAM, NL ISSN: 0966-6362, DOI: 10.1016/j.gaitpost.2015.03.005 * the whole document * | 1-20 | A61B |

-/--

The present search report has been drawn up for all claims

| Place of search | Date of completion of the search | Examiner |
|---|---|---|
| Berlin | 29 September 2017 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 16 2231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GALNA BROOK ET AL: "Accuracy of the Microsoft Kinect sensor for measuring movement in people with Parkinson's disease", GAIT & POSTURE, vol. 39, no. 4, 22 January 2014 (2014-01-22), pages 1062-1068, XP028836145, ISSN: 0966-6362, DOI: 10.1016/J.GAITPOST.2014.01.008 * the whole document *  ----- | 1-20 | |
| A | WO 2013/019956 A1 (CAPACITY SPORTS LLC [US]; CURTISS CHASE [US]) 7 February 2013 (2013-02-07) * page 6, line 3 - page 7, line 4 * * page 9, line 20 - page 10, line 30 *  ----- | 1-20 | |

TECHNICAL FIELDS
SEARCHED      (IPC)

The present search report has been drawn up for all claims

| Place of search | Date of completion of the search | Examiner |
|---|---|---|
| Berlin | 29 September 2017 | Pohjamo, Terhi |

EPO FORM 1503 03.82 (P04C01)

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

**EP 3 298 955 A1**

## ANNEX TO THE EUROPEAN SEARCH REPORT
## ON EUROPEAN PATENT APPLICATION NO.

EP 17 16 2231

This annex lists the patent family members relating to the patent documents cited in the above-mentioned European search report.
The members are as contained in the European Patent Office EDP file on
The European Patent Office is in no way liable for these particulars which are merely given for the purpose of information.

29-09-2017

| Patent document cited in search report | Publication date | Patent family member(s) | Publication date |
|---|---|---|---|
| WO 2013019956 A1 | 07-02-2013 | US 2013035613 A1<br>US 2017035343 A1<br>WO 2013019956 A1 | 07-02-2013<br>09-02-2017<br>07-02-2013 |

EPO FORM P0459

For more details about this annex : see Official Journal of the European Patent Office, No. 12/82

**EP 3 298 955 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 201621032626